# EUROPEAN PATENT SPECIFICATION

(11) **EP 0 591 329 B1**
(45) Date of publication and mention of the grant of the patent: **24.07.1996**
(21) Application number: 92913308.0
(22) Date of filing: 27.05.1992
(51) Int. Cl.: A61F 7/00, A61M 37/00, A61H 33/12

(54) **THE SKIN CLINIC STEAMER**
DAMPFGERÄT ZUR THERAPEUTISCHEN BEHANDLUNG DER HAUT
DISPOSITIF UTILISANT LES PROPRIETES THERAPEUTIQUES DE LA VAPEUR SUR L'EPIDERME

(30) Priority: 30.05.1991 US 707828
(43) Date of publication of application: 13.04.1994
(73) Proprietor: MEHL, Thomas L., Newberry, FL 32669 (US)
(72) Inventor: MEHL, Thomas L., Newberry, FL 32669 (US)
(74) Representative: Raeck, Wilfrid, Dipl.-Ing.
(86) International application number: US9204198
(87) International publication number: WO9221306

(56) References cited:
- GB-A- 2 095 550
- US-A- 4 596 565
- US-A- 4 597 757
- US-A- 4 657 531

## Description

### Field of the Invention

This invention relates to a new apparatus for using the therapeutic properties of steam by its application to the human body.

### Background of the Invention

From US-A-4 596 565 an applicator device is known, which facilitates applying of salve-like or liquid medicants to a body portion by using a stream of heated air. This device comprises a portable hand-held casing including a blower motor, heating wires, and an exit opening covered by a porous element, which cooperates with a covering fabric material impregnated with the medicant and providing a cushioned surface for applying to a body portion. The casing also houses a vibrating element operable to essentially vibrate the casing and thereby facilitate the absorption of the medicant into the body portion.

Further, a therapeutic heating apparatus known from US-A-4 657 531 may be utilized for treating human body portions by heated vapors produced from pharmaceutical ingredients. This apparatus comprises a casing to be hand held by a handle and including an electrical heater, a heat conducting plate and a cotton pad containing medicinal ingredients, which is kept engaged with the heat conducting plate by an outer cotton cover for vaporizing the ingredients of the pad and for making penetrate them into the body of the patient.

Steam has been used in body treatment because of the advantageous effect of moisture-laden vapor in skin and hair treatment and also as an inhalant. The use of steam in hair treatment allows hair fibers to be moistened and facilitates setting and quick drying of the hair. Steam as an inhalant allows water vapors to be drawn into the nasal passages and to moisturize those areas without blocking the air passages. Using steam in skin treatment is known generally and applied at steam baths causing perspiration, opening of the pores, and aids in relaxing muscles.

GB-A-2 095 550 which forms the preamble of claim 1 discloses a steamer device for treating skin surfaces, comprising a hand held housing, a steam generator disposed within said housing for supplying steam to the skin surfaces to be treated, and drive means disposed within said housing for propelling steam from said steam generator to the skin surfaces to be treated (preamble of claim 1). The housing of this portable sauna is shaped like a round flask with a wide bottom to ensure good stability. Its lower portion includes a water tank and a heating element, while its detachable upper portion shaped to a slanted elongated neck contains at its top an annular steam outlet. Exchangeable attachments such as a facial mask, concentrator tube, brush- or sponge-attachment can be mounted to the top of the upper housing portion and serve to regulate the temperature of the air-steam mixture.

Steam also can be used to support skin cleaning treatment, it helps to cleanse the skin through the heating of the natural oils to a more liquified state which facilitates removal through wiping or washing them away. In using steam as a skin treatment, certain areas of the body require more treatment than others. As, for example, the face is a most often treated area, it would be more convenient to have a steam applicator which can be applied to portions of the anatomy and allows for a smaller steam application device and a lower quantity of steam.

In view of the foregoing, it can be seen that there is a need for a device which uses the beneficial properties of steam to enhance effective body treatment. Therefore, the invention has for object to provide a device using steam in skin treatment and additionally being suited for contacting and buffing the skin in order to both intensify cleaning effects and relaxing muscles.

### Features and Summary of the Invention

To achieve this object, the hand held steamer device according to claim 1 of the invention comprises a rotating buffing head operatively connected to said drive means, and a porous pad removably secured to said buffing head and associated with said steam generator for simultaneously treating the skin surfaces as steam is applied to the skin surfaces from said steam generator.

According to improvements of the invention the pad may be formed of a natural fiber material or a filament fiber material or an abrasive material or a soft absorbent material.

According to further improvements the pad may include medication applied thereto, or the steam generator may include a supply of water and medication for supplying medicated steam to the skin surfaces.

In a further improvement the drive means includes a motorized fan for propelling steam from said steam generator to the skin surface to be treated. Said fan may include a rotating disc, and said buffing head may be removably mounted to said disc.

To improve the invention, the buffing head can be connected to the rotating disc by resiliently engaging the side edge of the rotating disc with extending legs.

Another embodiment of the invention resides in a buffing head including a porous surface for allowing the passage of steam therethrough.

According to another embodiment the pad is removably secured to said buffing head using hook and loop fastener material, or using a split ring.

According to a further aspect the buffing head includes a side wall having a plurality of hooks extending therefrom and said pad includes openings for engaging said hooks along said side wall.

The invention may also be improved by a pad operably associated with said steam generator such that the steam passes through said pad for simultaneously buffing and steaming the skin surfaces. Thus, the inventional device provides for a heating pad for applying steam and heat to portions of the body.

Preferably, the inventional steamer device comprises a water chamber with a heating element inside being removably secured to said housing. Further, the steamer device may comprise within the housing an autonomous power supply for the drive means, and the buffing head is motorized to provide a reciprocating or oscillating or vibrating action.

According to another embodiment the housing may include a handle which is formed between a finger hole and an outer wall of the housing.

Another embodiment of the invention resides in a water chamber forming a removable container being frictionally connected to the housing.

In summary, the invention is directed to a body treatment device which operates directly in contact with the skin and uses steam to enhance the effectiveness of the treatment. The specific treatment appliance can be realized as a buffing head which is motorized to provide a reciprocating or oscillating action to vigorously rub the skin surface to remove oils, and dead skin cells, make-up, dirt, and the like from the skin surface as steam is applied. A treated pad may be used on the applicator surface to directly apply medication to the skin. Medication may also be dissolved in the liquid and released in the steam vapor to come into contact with the skin. In using the inventional body treatment device a plurality of interchangeable buffing heads may be provided for efficiently applying a proper buffing head to varied contours of the body.

The invention will be further described by embodiments shown in the drawings.

### Brief Description of the Drawings

Figure 1 is a perspective view of a hand held skin steaming and buffing apparatus according to the invention;
Figure 2 is a cross-sectional view of the apparatus shown in Fig. 1;
Figure 3 shows a self-contained steaming and buffing apparatus according to the invention;
Figure 4 is a plan view of a cross-section of the buffing head taken along lines 4-4 in Fig. 3;
Figure 5 is a side view of the buffing head, while
Figures 6 - 9 are different embodiments showing different buffing head shapes and pad attachment structures.

### Detailed Description of the Invention

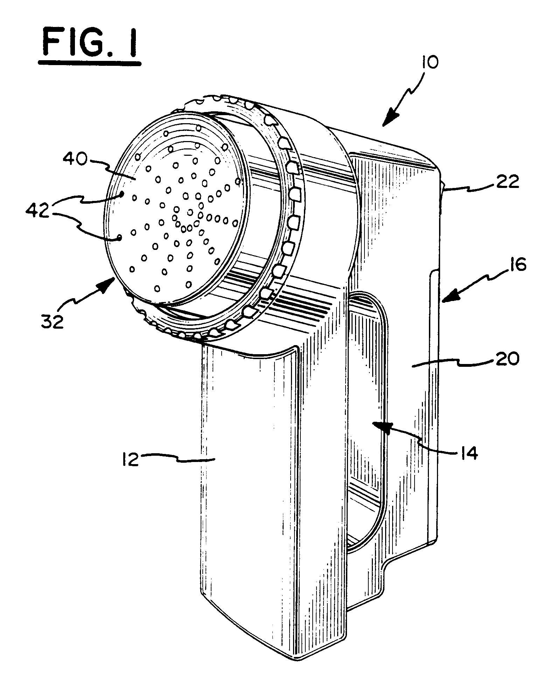

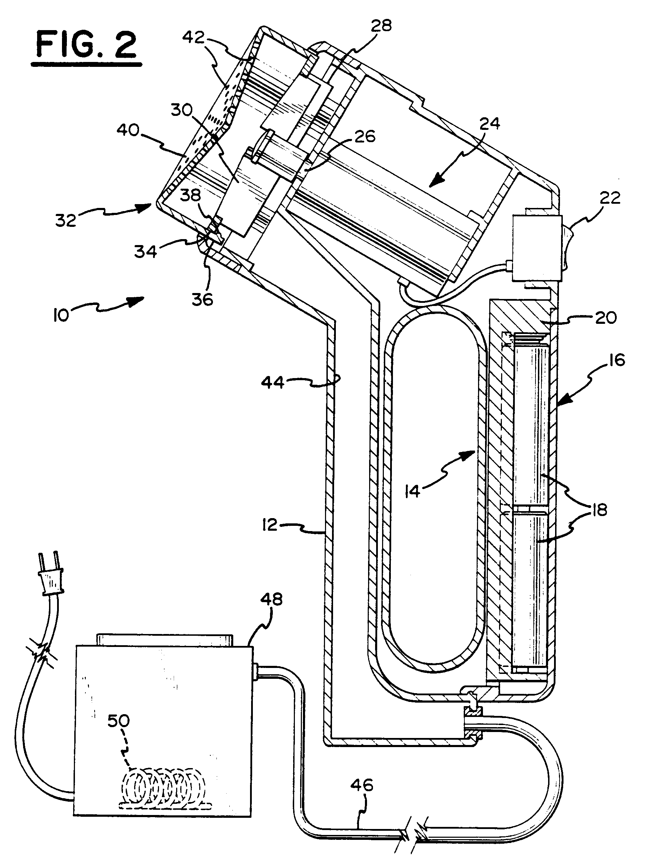

Figure 1 and Fig. 2 show a skin treatment device 10 having an exterior casing 12 formed of plastic or other suitable material. Plastic is preferred for its durability and light weight. Housing 12 includes a finger opening 14 therein which permits grasping of the housing 12 by the hand so that the fingers may wrap around through the hole 14 and grasp handle portion 16.

Inside the handle portion 16 is located a set of batteries 18 located within a formed housing 20 and includes the conventional wiring for providing electricity from the batteries 18 to the switch 22. The batteries are accessible through a removable plate (not shown). It should be understood that a rechargeable battery or other power source could be substituted for the batteries 18.

Switch 22 is preferably simply an on-off device, but could also be a rheostat for variably supplying electric current from the batteries 18 to a motor 24.

Motor 24 is held in axial alignment with respect to the upper portion of the housing 12. Motor 24 is preferably of sufficient power to rotate a drive shaft at a speed between 20 and 80 rpm. Motor 24 also has sufficient torque to drive its shaft through a certain amount of resistance. The motor includes a drive shaft 26 which is connected to a rotating disk 28.

Preferably, rotating disk 28 includes a plurality of fan blades 30 which assist in sweeping the steam laden vapor out of the housing 12 and upward to a buffing head 32. Buffing head 32 is removably connected to the rotating disk 28 by resiliently engaging the side edge 34 of the rotating disc 28 with extending legs 36 having detents 38 for engaging the side edge 34 resiliently.

Figure 1 shows the buffing head 32 having a concave face 40, but other shapes may be used depending on the contour of the particular area of the body. Face 40 of buffing head 32 includes a plurality of perforations 42 which permit vapor to pass therethrough.

Housing 12 includes a steam passageway 44 which connects to a hose 46 extending from a steam generator 48. The steam generator 48 includes a resistor element 50 which is supplied with electricity from a conventional plug-in outlet (not shown). The resistor element 50 heats water in the steam chamber to create the steam which flows along hose 46 and into the housing 12. The steam generator 48 is preferably large enough to hold about a pint of water. The water may be inserted through a screw-on cap (not shown).

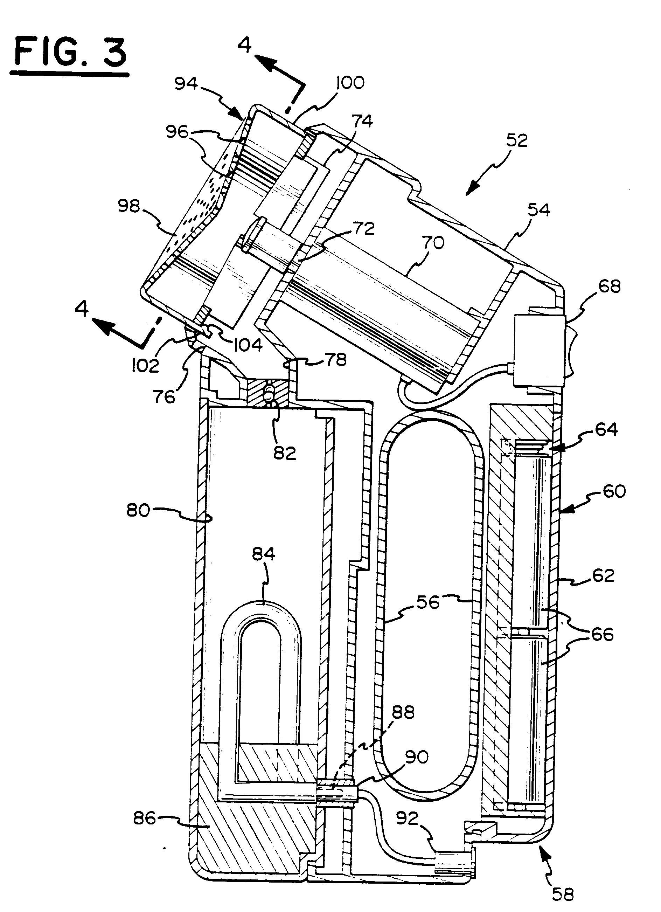

Figure 3 shows a skin treatment device 52 which is a self-contained unit. The unit has a housing 54 having a handle hole 56 formed therein. A handle 58 is formed between the hole 56 and an outer wall 60 of the housing 54.

Wall 60 includes a removable door 62 which is removeably attachable to wall 60 to provide access to a battery chamber 64. A pair of batteries 66 are normally located within the battery chamber 64 and are preferably a pair of AA batteries connected in series which provide power to a switch 68. Switch 68 is preferably simply an on-off device, however a rheostat may also be substituted to provide a variable control over electrical energy flowing to the motor 70.

Motor 70 is designed to operate in an rpm range between 20 and 80 rpm preferably, and provides sufficient torque to a drive shaft 72 to overcome a predetermined degree of resistance. Drive shaft 72 is connected to a combination disc and fan assembly 74 which performs two functions. First, the fan assembly 74 draws air through opening 76 from the exterior of the housing 54 and also mixes this air with steam flowing through passageway 78 from water chamber 80.

Water is prevented from flowing from water chamber 80 into passageway 78 when the skin treatment device 52 is inverted by one-way valve 82 which permits steam to pass therethrough but prevents the passage of water.

Water chamber 80 forms a container which is removable from the skin treatment device 52 and frictionally connects to housing 54.

Water chamber 80 includes a resistor element 84 which heats the water held therein to generate steam. Resistor element 84 is held in place by a suitable resin 86 and includes a through wall fitting 88 which is connectable electrically to a female receptacle 90 connected to housing 54. Female receptacle 90 is electrically connected to another female receptacle 92 which is connectable with an electric cord for connecting the receptacle 92 to a wall socket.

As steam is generated in the water chamber 80, the steam flows through one-way valve 82 into passageway 78. The steam is then mixed with incoming air from passageway 76 and is mixed and drawn by fan 74 to cool the steam and propel the steam/air mixture through buffing head 94. Buffing head 94 includes a series of passageways 96 in concave face 98.

A circumferential side wall 100 extends downwardly from face 98 and preferably includes three extending legs 102 having detents 104 thereon for engaging the rotating disc assembly 74 and maintaining the buffing head 94 in proper position.

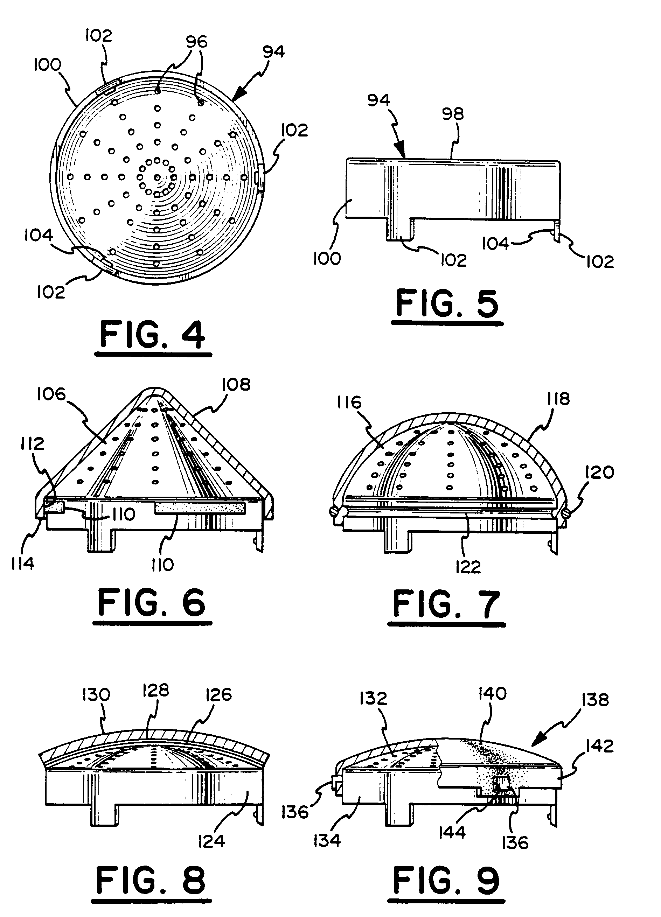

A section taken along lines 4-4 of Figure 3 is shown in Figure 4 of the buffing head 94. Figure 4 shows the pattern arrangement of perforations 96 preferably used in the buffing head. Figure 5 shows the side wall 100 having depending legs 102 and detents 104. Preferably, buffing head 94 is sized to completely cover the disk and fan assembly 74 to prevent the escape of steam along the side wall 100 and enhance the flow of steam through the perforations 96. If the buffing head 94 is smaller than the area covered by the disc and fan assembly 74, then steam will escape around the side wall 100 which may result in a loss of effectiveness.

Figure 6 shows a conical shaped buffing head 106 having a pad 108 mounted thereon. Pad 108 may be formed of slightly abrasive material which aids in removing dirt and make-up and dead skin cells from the skin surface. Pad 108 may also be made of an absorbent material such as cotton to hold medication or absorb oils from the skin. Pad 108 may also be made of natural or filament fiber material. Commercially available pads may be used such as Stridex™ brand. Pad 108 is held in position by a series of hook and loop fastener material. The hook and loop fastener material includes one set of hooks or loops 110 mounted on the buffing head 106 and a second set of hooks or loops mounted on the inside surface 112 near the pad edge 114. The conical shape of buffing head 106 is preferred for areas of the skin such as the sides of the nose and around the mouth for maximum contact between the pad 106 and the skin surface.

Figure 7 shows a hemispherical shaped head 116 having a pad 118 mounted thereon. Pad 118 is held in place on head 116 by a snap ring 120 which clips into a circular slot 122 below the hemispherical head 116. Ring 120 preferably forms an arc of between 180° and 360°.

Figure 8 shows a curved buffing head 124 which is covered with hook and loop fastener material 126. Pad 128 includes complimentary hook and loop fastener material 130 mounted to the under surface thereof to connect with the hook and loop fastener surface 126 on the buffing head 124. Pad 128 may also be constructed of material which is complimentarily fastenable to the hook and loop fasteners 126.

Figure 9 shows a curved head 132 having along a side wall 134 a set of pad-engaging hooks 136. Pad 138 includes a curved surface 140 covering the curved head 132 and includes a side wall 142 surrounding a portion of the side wall 134 of the buffing head 132. Side wall 142 includes openings 144 for engaging hooks 136 on side wall 134 for retaining the pad 138 on curved buffing head 132.

While this invention has been described as having a preferred design, it is understood that it is capable of further modifications, and uses and/or adaptations of the invention and following in general the principle of the invention and including such departures form the present disclosure as come within the known or customary practice in the art to which the invention pertains, and in any case be applied to the central features hereinbefore set forth, and fall within the scope of the claims appended hereto.

### List of Reference Signs

- **Fig. 1 - 2:**:
- 10: - skin treatment device
- 12: - exterior housing
- 14: - finger opening
- 16: - grasp handle portion
- 18: - batteries
- 20: - formed housing (for batteries 18)
- 2: 2 - switch
- 24: - motor
- 26: - drive shaft
- 28: - rotating disc
- 30: - fan blades
- 32: - buffing head
- 34: - side edge
- 36: - extending leges
- 38: - detents
- 40: - concave face
- 42: - perforations
- 44: - steam passageway
- 46: - hose
- 48: - steam generator
- 50: - resistor element
- **Fig. 3 - 5:**:
- 52: - skin treatment device
- 54: - housing
- 56: - handle hole
- 60: - outer wall
- 62: - removable door
- 64: - battery chamber
- 66: - pair of batteries
- 68: - switch
- 70: - motor
- 72: - drive shaft
- 74: - disc and fan assembly
- 76: - opening
- 78: - passageway
- 80: - water chamber
- 82: - one-way valve
- 84: - resistor element
- 86: - resin
- 88: - through wall fitting
- 90: - female receptacle
- 92: - another female receptable
- 94: - buffing head
- 96: - passageways
- 98: - concave face
- 100: - circumferencial side wall
- 102: - extending legs
- 104: - detents
- **Fig. 6:**:
- 106: - conical shaped buffing head
- 108: - pad
- 110: - set of hook and loops
- 112: - inside surface
- 114: - pad edge
- **Fig. 7:**:
- 116: - hemispherical shaped buffing head
- 118: - pad
- 120: - snap ring
- 122: - circular slot
- **Fig. 8:**:
- 124: - curved buffing head
- 126: - hook and loop fastener material
- 128: - pad
- 130: - complimentary hook and loop fastener material
- **Fig. 9:**:
- 132: - curved buffing head
- 134: - side wall
- 136: - pad-engaging hooks
- 138: - pad
- 140: - curved surface
- 142: - side wall
- 144: - openings

## Claims

1. A hand held steamer device for treating skin surfaces, comprising a hand held housing (12; 54), a steam generator (48) disposed within said housing (12; 54) for supplying steam to the skin surfaces to be treated, and drive means (24;70) disposed within said housing (12; 54) for propelling steam from said steam generator (48) to the skin surfaces to be treated,
**characterized by**
a rotating buffing head (32; 94; 106; 116; 124;132) removably connected to said drive means (24; 70), and
a porous pad (108; 118; 128; 138) removably secured to said rotating buffing head (32; 94; 106; 116; 124; 132) for simultaneously treating the skin surfaces as steam is applied to the skin surfaces from said steam generator (48).

2. The hand held steamer device as in claim 1, wherein said pad (108; 118; 128; 138) is formed of a natural fiber material.

3. The hand held steamer device as in claim 1 or 2, wherein said pad (108; 118; 128; 138) is formed of a filament fiber material.

4. The hand held steamer device as in one of the preceding claims, wherein said pad (108; 118; 128; 138) is formed of or includes an abrasive material.

5. The hand held steamer device as in one of the preceding claims, wherein said pad (108; 118; 128; 138) is formed of a soft absorbent material.

6. The hand held steamer device as in one of the preceding claims, wherein said pad (108; 118; 128; 138) includes medication applied thereto.

7. The hand held steamer device as in one of the preceding claims, wherein said steam generator (48) includes a supply of water and medication for supplying medicated steam to the skin surfaces.

8. The hand held steamer device as in one of the preceding claims, wherein said drive means (24; 70) further includes a motorized fan (30; 74) for propelling steam from said steam generator (48) to the skin surface to be treated.

9. The hand held steamer device as in claim 8, wherein said fan (30; 74) includes a rotating disc (28; 74), and said buffing head (32; 94; 106; 116; 124; 132) is removably mounted to said disc (28; 74).

10. The hand held steamer device as in claim 9, wherein said buffing head (32; 94; 106; 116; 124; 132) is connected to the rotating disc (28; 74) by resiliently engaging the side edge (34) of the rotating disc (28; 74) with extending legs (36; 102).

11. The hand held steamer device as in one of the preceding claims, wherein said buffing head (32; 94; 106; 116; 124; 132) includes a porous surface for allowing the passage of steam there-through.

12. The hand held steamer device as in one of the preceding claims, wherein said pad (108; 118; 128; 138) is removably secured to said buffing head (32; 94; 106; 116; 124; 132) using hook and loop fastener material (110; 126; 130).

13. The hand held steamer device as in one of the preceding claims, wherein said pad (108; 118; 128; 138; 152) is removably secured to said buffer using a split ring (120).

14. The hand held steamer device as in one of the preceding claims, wherein said buffing head (132) includes a side wall (134) having a plurality of hooks (136) extending therefrom and said pad (138) includes openings (144) for engaging said hooks (136) along said side wall (134).

15. The hand held steamer device as in one of the preceding claims, wherein said pad (108; 118; 128; 138) operably associated with said steam generator (48) such that the steam passes through said pad (108; 118; 128; 138; 152) for simultaneously buffing and steaming the skin surfaces.

16. The hand held steamer device as in one of the preceding claims, wherein a water chamber (80) with a heating element (84) inside is removably secured to said housing (12,54).

17. The hand held steamer device as in one of the preceding claims, wherein said housing (12; 54) comprises an autonomous power supply (18; 66) for the drive means (24; 70).

18. The hand held steamer device as in one of the preceding claims, wherein the housing (12; 54) includes a handle (16; 58) which is formed between a finger hole (14; 56) and an outer wall (60) of the housing.

19. The hand held steamer device as in one of the preceding claims, wherein a water chamber (80) forming a removable container is frictionally connected to the housing (12; 54).

## Patentansprüche

1. Handgehaltenes Dampfgerät zur Behandlung von Hautoberflächen, das ein handgehaltenes Gehäuse (12; 54), einen darin angeordneten Dampferzeuger (48), zur Dampfzuführung zu den zu behandelnden Hautoberflächen, und im Gehäuse (12; 54) angeordnete Antriebsmittel (24; 70) umfaßt, um Dampf vom Dampferzeuger (48) den zu behandelnden Hautoberflächen zuzutreiben, **gekennzeichnet durch**
einen mit den Antriebsmitteln (24; 70) abnehmbar verbundenen drehenden Poliermassage-Kopf (32; 94; 106; 116; 124; 132) und
einem am drehenden Poliermassage-Kopf (32; 94; 106; 116; 124; 132) abnehmbar befestigten porösen Polsterbelag (108; 118; 128; 138), der die Hautoberflächen gleichzeitig behandelt, während der Dampf vom Dampfgenerator (48) den Hautoberflächen zugeführt wird.

2. Handgehaltenes Dampfgerät nach Anspruch 1, dadurch gekennzeichnet, daß der Polsterbelag (108; 118; 128; 138) aus einem natürlichen Faser-Material gebildet ist.

3. Handgehaltenes Dampfgerät nach Anspruch 1 oder 2, dadurch gekennzeichnet, daß der Polsterbelag (108; 118; 128; 138) aus einem Fadenfaser-Material gebildet ist.

4. Handgehaltenes Dampfgerät nach einem der vorhergehenden Ansprüche, dadurch gekennzeichnet, daß der Polsterbelag (108; 118; 128; 138) aus einem abreibenden Material gebildet ist oder ein solches enthält.

5. Handgehaltenes Dampfgerät nach einem der vorhergehenden Ansprüche, dadurch gekennzeichnet, daß der Polsterbelag (108; 118; 128; 138) aus einem weichen saugfähigen Material gebildet ist.

6. Handgehaltenes Dampfgerät nach einem der vorhergehenden Ansprüche, dadurch gekennzeichnet, daß der Polsterbelag (108; 118; 128; 138) darauf aufgebrachte Medikationsmittel enthält.

7. Handgehaltenes Dampfgerät nach einem der vorhergehenden Ansprüche, dadurch gekennzeichnet, daß der Dampferzeuger (48) einen Vorrat an Wasser und Medikationsmitteln enthält, um den Hautoberflächen mit Medikationsmitteln versetzten Dampf zuzuführen.

8. Handgehaltenes Dampfgerät nach einem der vorhergehenden Ansprüche, dadurch gekennzeichnet, daß die Antriebsmittel (24; 70) außerdem ein motorisch angetriebenes Gebläse (30; 74) enthalten, um Dampf vom Dampferzeuger (48) zu der zu behandelnden Hautoberfläche zu treiben.

9. Handgehaltenes Dampfgerät nach Anspruch 8, dadurch gekennzeichnet, daß das Gebläse (30; 74) eine drehende Scheibe (28; 74) aufweist und der Poliermassagekopf (32; 94; 106; 116; 124; 132) an dieser Scheibe (28; 74) abnehmbar angebracht ist.

10. Handgehaltenes Dampfgerät nach Anspruch 9, dadurch gekennzeichnet, daß der Poliermassagekopf (32; 94; 106; 116; 124; 132) durch längliche Stege (36; 102) mit der drehenden Scheibe (28; 74) verbunden ist, die mit der Seitenkante (34) der drehenden Scheibe (28; 74) in federndem Eingriff stehen.

11. Handgehaltenes Dampfgerät nach einem der vorhergehenden Ansprüche, dadurch gekennzeichnet, daß der Poliermassagekopf (32; 94; 106; 116; 124; 132) eine den Durchgang von Dampf gewährende poröse Fläche aufweist.

12. Handgehaltenes Dampfgerät nach einem der vorhergehenden Ansprüche, dadurch gekennzeichnet, daß der Polsterbelag (108; 118; 128; 138) unter Verwendung von Haken und Ösen aufweisendem Befestigungsmaterial (110; 126; 130) am Massagekopf (32; 94; 106; 116; 124; 132) abnehmbar befestigt ist.

13. Handgehaltenes Dampfgerät nach einem der vorhergehenden Ansprüche, dadurch gekennzeichnet, daß der Polsterbelag (108; 118; 128; 138) unter Verwendung eines geschlitzten Ringes (120) abnehmbar am Massagekopf (32; 94; 106; 116; 124, 132) befestigt ist.

14. Handgehaltenes Dampfgerät nach einem der vorhergehenden Ansprüche, dadurch gekennzeichnet, daß der Poliermassagekopf (132) eine Seitenwand (134) mit einer Mehrzahl davon abstehender Haken (136) aufweist, und daß der Polsterbelag (138) Öffnungen (144) enthält, die entlang der Seitenwand (134) mit den Haken (136) in Eingriff bringbar sind.

15. Handgehaltenes Dampfgerät nach einem der vorhergehenden Ansprüche, dadurch gekennzeichnet, daß der Polsterbelag (108; 118; 128; 138) derart mit dem Dampferzeuger (48) zusammenwirkt, daß der Dampf das Polster (108; 118; 128; 138) durchströmt, um die Hautoberflächen gleichzeitig zu bedampfen und zu massieren.

16. Handgehaltenes Dampfgerät nach einem der vorhergehenden Ansprüche, dadurch gekennzeichnet, daß am Gehäuse (12; 54) eine ein Heizelement (84) enthaltende Wasserkammer (80) abnehmbar befestigt ist.

17. Handgehaltenes Dampfgerät nach einem der vorhergehenden Ansprüche, dadurch gekennzeichnet, daß das Gehäuse (12; 54) mit einer autonomen Stromversorgung (18; 66) für die Antriebsmiftel (24; 70) versehen ist.

18. Handgehaltenes Dampfgerät nach einem der vorhergehenden Ansprüche, dadurch gekennzeichnet, daß das Gehäuse (12; 54) einen Handgriff (16; 58) aufweist, der zwischen einer Fingeröffnung (14; 56) und einer Außenwand (60) des Gehäuses (12; 54) gebildet ist.

19. Handgehaltenes Dampfgerät nach einem der vorhergehenden Ansprüche, dadurch gekennzeichnet, daß eine einen abnehmbaren Behälter bildende Wasserkammer (80) mit dem Gehäuse reibkraftschlüssig(12; 54) verbunden ist.

## Revendications

1. Dispositif générateur de vapeur à main pour traiter des surfaces de la peau, comprenant un boîtier à main (12 ; 54), un générateur de vapeur (48) monté à l'intérieur dudit boîtier (12 ; 54) pour appliquer de la vapeur sur les surfaces de la peau à traiter, et des moyens d'entraînement (24 ; 70) montés dans ledit boîtier (12 ; 54) pour propulser la vapeur formée par ledit générateur de vapeur (48) vers les surfaces de la peau à traiter, caractérisé par :
une tête à polir rotative (32 ; 94 ; 106 ; 116 ; 124 ; 132) montée amovible sur lesdits moyens d'entraînement (24 ; 70), et
un bloc poreux (108 ; 118 ; 128 ; 138) fixé amovible sur ladite tête rotative à polir (32 ; 94 ; 106 ; 116 ; 124 ; 132) pour traiter simultanément les surfaces de la peau lorsque de la vapeur provenant dudit générateur de vapeur (48) est appliquée sur les surfaces de la peau.

2. Dispositif manuel à vapeur selon la revendication 1, dans lequel ledit bloc (108 ; 118 ; 128 ; 138) est formé d'un matériau de fibres naturelles.

3. Dispositif manuel à vapeur selon la revendication 1 ou 2, dans lequel ledit bloc (108 ; 118 ; 128 ; 138) est formé d'un matériau de fibres à filament.

4. Dispositif manuel à vapeur selon l'une des revendications précédentes, dans lequel ledit bloc (108 ; 118 ; 128 ; 138) est formé de ou contient un matériau abrasif.

5. Dispositif manuel à vapeur selon l'une des revendications précédentes, dans lequel ledit bloc (108 ; 118 ; 128 ; 138) est formé d'un matériau doux absorbant.

6. Dispositif manuel à vapeur selon l'une des revendications précédentes, dans lequel ledit bloc (108 ; 118 ; 128 ; 138) contient un médicament qui lui est appliqué.

7. Dispositif manuel à vapeur selon l'une des revendications précédentes, dans lequel ledit générateur de vapeur (48) comprend une réserve d'eau et un médicament afin d'appliquer de la vapeur contenant un médicament sur les surfaces de la peau.

8. Dispositif manuel à vapeur selon l'une des revendications précédentes, dans lequel lesdits moyens d'entraînement (24 ; 70) comprennent en outre un ventilateur à moteur (30 ; 74) pour propulser la vapeur formée par ledit générateur de vapeur (48) vers la surface de la peau a traiter.

9. Dispositif manuel à vapeur selon la revendication 8, dans lequel ledit ventilateur (30 ; 74) comprend un disque rotatif (28 ; 74), et ladite tête à polir (32 ; 94 ; 106 ; 116 ; 124 ; 132) est montée amovible sur ledit disque (28 ; 74).

10. Dispositif manuel à vapeur selon la revendication 9, dans lequel ladite tête à polir (32 ; 94 ; 106 ; 116 ; 124 ; 132) est reliée au disque rotatif (28 ; 74) en mettant en prise de manière élastique le bord latéral (34) du disque rotatif (28 ; 74) avec des pattes qui dépassent (36 ; 102).

11. Dispositif manuel à vapeur selon l'une des revendications précédentes, dans lequel ladite tête à polir (32 ; 94 ; 106 ; 116 ; 124 ; 132) comprend une surface poreuse pour permettre le passage de la vapeur à travers.

12. Dispositif manuel à vapeur selon l'une des revendications précédentes, dans lequel ledit bloc (108 ; 118 ; 128 ; 138) est fixé amovible sur ladite tête à polir (32 ; 94 ; 106 ; 116 ; 124 ; 132) en utilisant un crochet et un matériau à boucle de fixation (110 ; 126 ; 130).

13. Dispositif manuel à vapeur selon l'une des revendications précédentes, dans lequel ledit bloc (108 ; 118 ; 128 ; 138 ; 152) est fixé amovible sur ladite tête à polir au moyen d'un anneau fendu (120).

14. Dispositif manuel à vapeur selon l'une des revendications précédentes, dans lequel ladite tête à polir (132) comprend une paroi latérale (134) de laquelle dépasse une pluralité de crochets (136) et dans lequel ledit bloc (138) comprend des ouvertures (144) destinées à venir en prise avec lesdits crochets (136) le long de ladite paroi latérale (134).

15. Dispositif manuel à vapeur selon l'une des revendications précédentes, dans lequel ledit bloc (108 ; 118 ; 128 ; 138) est associé fonctionnellement avec ledit générateur de vapeur (48) de manière que la vapeur traverse ledit bloc (108 ; 118 ; 128 ; 138 ; 152) pour simultanément polir les surfaces de la peau et les passer à la vapeur.

16. Dispositif manuel à vapeur selon l'une des revendications précédentes, dans lequel une chambre à eau (80) dans lequel est installé un élément chauffant (84) est fixée amovible sur ledit boîtier (12, 54).

17. Dispositif manuel à vapeur selon l'une des revendications précédentes, dans lequel ledit boîtier (12 ; 54) comprend une source d'alimentation autonome (18 ; 66) pour les moyens d'entraînement (24 ; 70).

18. Dispositif manuel à vapeur selon l'une des revendications précédentes, dans lequel le boîtier (12 ; 54) comprend une poignée (16 ; 58) qui est formée entre un orifice destiné à recevoir les doigts (14 ; 56) et une paroi extérieure (60) du boîtier.

19. Dispositif manuel à vapeur selon l'une des revendications précédentes, dans lequel une chambre à eau (80) constituant un réservoir amovible est relié au boîtier (12 ; 54) par friction.
